# EUROPEAN PATENT SPECIFICATION

(11) **EP 3 050 865 B1**
(45) Date of publication and mention of the grant of the patent: **24.02.2021**
(21) Application number: 15202925.2
(22) Date of filing: 29.12.2015
(51) Int. Cl.: C07C 1/12, C07C 9/04, B01J 23/83, B01J 37/34

(54) **PROCESS FOR THE REDUCTION OF CARBON DIOXIDE TO METHANE BY DBD PLASMA-ACTIVATED CATALYST**
VERFAHREN ZUR REDUKTION VON KOHLENDIOXID IN METHAN DURCH DBD-PLASMAAKTIVIERTEN KATALYSATOR
PROCÉDÉ POUR LA RÉDUCTION DE DIOXYDE DE CARBONE EN MÉTHANE PAR UN CATALYSEUR DBD ACTIVÉ PAR PLASMA

(30) Priority: 28.01.2015 ES 201530109
(43) Date of publication of application: 03.08.2016
(73) Proprietor: Sorbonne Université, 75006 Paris (FR); École Nationale Superieure de Chimie de Paris - Chimie ParisTech, 75005 Paris (FR); Paris Sciences et Lettres - Quartier Latin, 75006 Paris (FR)
(72) Inventor: ANDREU ARBELLA, Teresa, 08930 Sant Adrià de Besòs (ES); MORANTE LLEONART, Juan Ramón, 08028 Barcelona (ES); AMOUROUX, Jacques, 91440 Bures S/ Yvette (FR); CAVADIAS, Simeon, 75005 Paris (FR); NIZIO, Magdalena, 75252 Paris Cedex 05 (FR); OGNIER, Stéphanie, 75005 PARIS (FR); FARIA DE BARROS HENRIQUES, Carlos Manuel, 1049-001 Lisboa (PT); MADEIRA LOPES, José Manuel Felix, 1049-001 Lisboa (PT); BISCAYA SEMEDO PEREIRA DA GRAÇA, Inês Sofia, 1049-001 Lisboa (PT); GOMES RIBEIRO, Maria Filipa, 1049-001 Lisboa (PT); BACARIZA REY, María del Carmen, 1049-001 Lisboa (PT)
(74) Representative: Ponti & Partners, S.L.P

(56) References cited:
- DE-A1- 4 220 865
- DATABASE WPI Section Ch, Week 201121 Thomson Scientific, London, GB; Class A97, AN 2010-Q15506 XP002758136, -& CN 101 880 214 A (UNIV DALIAN TECHNOLOGY) 10 November 2010 (2010-11-10)
- SOUDABEH RAHMANI ET AL: "Preparation of promoted nickel catalysts supported on mesoporous nanocrystalline gamma alumina for carbon dioxide methanation reaction", JOURNAL OF INDUSTRIAL AND ENGINEERING CHEMISTRY, vol. 20, no. 6, 1 November 2014 (2014-11-01), pages 4176-4182, XP055275911, KOREA ISSN: 1226-086X, DOI: 10.1016/j.jiec.2014.01.017
- DATABASE WPI Week 201304 Thomson Scientific, London, GB; AN 2012-N56296 XP002758137, -& CN 102 600 854 A (UNIV SICHUAN) 25 July 2012 (2012-07-25)
- HEZHI LIU ET AL: "Effect of CeO2 addition on Ni/Al2O3 catalysts for methanation of carbon dioxide with hydrogen", JOURNAL OF NATURAL GAS CHEMISTRY., vol. 21, no. 6, 1 November 2012 (2012-11-01), pages 703-707, XP055276014, US, CN ISSN: 1003-9953, DOI: 10.1016/S1003-9953(11)60422-2

## Description

### Field of the invention

The present invention relates to the field of synthetic production of methane. In particular, the present invention relates to a low temperature process which uses a catalyst activated by a DBD (dielectric barrier discharge) plasma.

### Background Art

According to the Sabatier Reaction, the reduction of carbon dioxide is performed by the interaction with hydrogen on, for example, Ni based catalysts and the obtained products are CH₄ + H₂O. The reaction is as follows:

CO₂+ 4 H₂ => CH₄ + 2 H₂O

(ΔH = - 165 KJ/mol)

Up to date, this process can be carried out at low temperatures (T<250°C) but the conversion is only close to zero %.

In addition, if this process is performed at high temperature (T>420°C) this process can lead to a secondary reaction due to the exothermicity of the process itself in which the methane selectivity decreases and the products are transformed into CO + H₂.

Therefore, there still exists the need for a process for obtaining methane as shown above in which the process can be carried out at low temperatures and atmospheric pressure with a high conversion rate (at least > 80%) and high selectivity (> 95%) and without any secondary reactions.

### Summary of the invention

The present inventors have unexpectedly found that the use of selected catalysts activated by a DBD plasma allows to overcome all the above mentioned drawbacks present in the typical Sabatier reaction and to perform the reaction at low temperatures with high yield.

The present invention relates to a process for performing the Sabatier reaction with a catalyst activated by a DBD (dielectric barrier discharge) plasma from room temperature up to those ranges in which CO+H₂ is formed.

The present disclosure also relates to a reactor suitable for performing the process according to the present invention.

### Brief description of the drawings

**Figure 1****.** Functional scheme of a DBD reactor. Plasma catalysis reactor for methanation of CO₂.

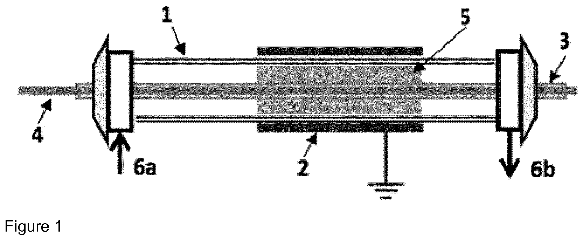

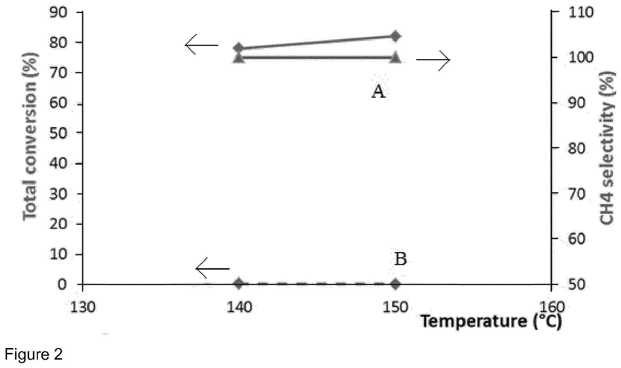

**Figure 2****.** Comparison between plasma and without plasma of the total CO₂ conversion and methane selectivity in the range of temperature around 150°C obtained using the DBD reactor described in figure 1 and the mesoporous catalyst developed on the base of a mesoporous SBA-15 replica of Ceria-Zirconia that has been impregnated with Nickel in a percentage of 15% of nickel with a composition of 10% of zirconia. Working procedure has been carried out under adiabatic conditions. In the figure, A (upper part of the representation) means with plasma and B (lower part of the representation) without plasma.

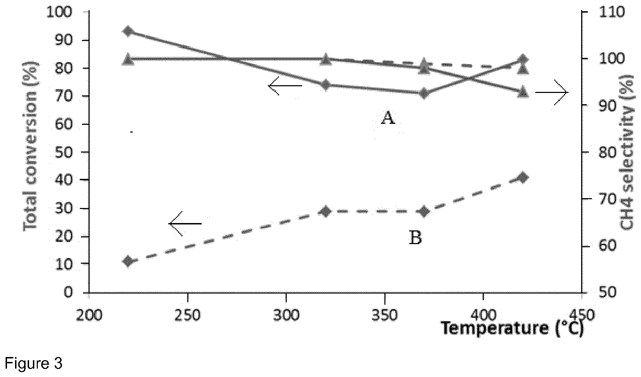

**Figure 3****.** Comparison between plasma and without plasma of the total CO₂ conversion and methane selectivity in the range standard of operation temperature obtained using the DBD reactor described in figure 1 and the mesoporous catalyst developed on the base of a mesoporous SBA-15 replica of Ceria-Zirconia that has been impregnated with Nickel in a percentage of 15% of nickel with a composition of 10% of zirconia. Working procedure has been carried out under isothermal conditions. In the figure, A (upper part of the representation) means with plasma and B (lower part of the representation) without plasma.
**Figure 4****.** High-resolution transmission electron microscopy of the mesoporous ceria-zirconia material impregnated with nickel showing the wall (figure 4A, left) and porous size of these nanostructured catalysts (figure 4B, right).
**Figure 5****.** Thermal programmed reduction with hydrogen of the mesoporous ceria zirconia material impregnated with nickel, corresponding the upper line to 15% Ni-CeZr (80:20), the central line to 15% Ni-CeZr (90:10) and the lower line to 15% Ni-Ce.
**Figure 6**. Hydrogen consumption under H2-TPR (temperature programmed reduction) conditions pointing that, after reductive pre-treatment, most of the Ni particles are reduced. The vertical line shows the pre-reduction temperature (470°C) being the area below the curve on the left (lower temperature) the main reduction for NiO.
**Figure 7** High resolution transmission electron microscopy (TEM) of the Ni zeolite-based catalysts, having figure 7A (upper figure) a lower resolution and figure 7B (lower figure) a higher resolution.

### Detailed description of the invention

The present invention relates to a process for performing the following reaction (Sabatier reaction) with a catalyst:

CO₂ + 4 H₂ -> CH₄ + 2H₂O

characterised in that:
- said catalyst is based on the combination of ceria and nickel; and
- said catalyst is activated by a DBD (dielectric barrier discharge) plasma.

Preferably, said catalyst based on the combination of ceria and nickel is obtained from a mesoporous ceria or ceria-zirconia enriched with nickel (Ni) or from a zeolite impregnated with Ni and ceria. The concentration of zirconia and nickel in the ceria allow to regulate the oxygen capacity storage as well as the own catalytic function. Furthermore, the use of a ternary support with variable composition modifies the electrical and thermal conductivity of the catalytic material which determines the stabilization temperature working under adiabatic conditions. The composition, structure and nickel content of the catalyst based on mesoporous structures or zeolites have influence on the adiabatic and isothermal ranges.

In the present invention, by "mesoporous" is meant a material containing pores with diameters between 2 and 50 nm

Due to the exothermic characteristics of the reaction, the nanostructure and composition of the catalysts have also influence on the kinetic rate of the reaction.

Due to fact that the measured temperatures in the process are that of the catalytic bed and not that of the catalytic site, the nature of the catalyst and its thermal stability is an important aspect in the process of the invention. This difference explains not only the appearance of the RWGS (Reverse Water Gas Shift) reaction at 350°C (measured temperature), but also the ageing of the catalyst and changes of the nature of the catalytic sites (carbon deposition, trapping of water in the form of hydrate or carbonate hydrate).

The suitability of catalyst resides in its intrinsic characteristics: its porosity and specific surface area, the nature of the metallic sites, the content of alkali, alkaline earth or rare earth ions (probably responsible for the ionic or electronic exchanges in the surface), the polarizability of adsorption sites, and the electrical and thermal conductivity of the substrate/support.

Likewise, another parameter to be considered for choosing the catalyst is the thermal conductivity of the support for controlling of the temperature of the catalytic bed.

The metallic sites such as nickel and some additional alkaline oxide or elements (Na, K, Ca, Mg, Cs,..) as well as rare earth oxides, control the kinetics and secondary reactions.

At the same time, the surface temperature and its polarization by the plasma need a specific electrical conductibility of the bulk. In this way, the instant process allows to control the kinetic adsorption and desorption of species and mainly of the H₂O.

The process of the invention binds the combined effect between ceria and nickel. It has been performed and proved using two types of approaches. The first one is based on a mesoporous ceria-zirconia composite usually obtained by a replication method of different types of mesoporous silica (SBA-15, KIT-6...) which is enriched with nickel. In a further embodiment, the mesoporous ceria or alternatively ceria-zirconia enriched with nickel also comprises rare-earth oxides. In a further preferred embodiment, the nickel content ranges from 5 to 15 wt% with respect to the whole composition of the catalyst and the zirconia content, in the case of ceria zirconia, up to 20 wt% forming a ternary ceria zirconia oxide compound. The second approach is based on a zeolite (in particular HY zeolite) impregnated with Ni and ceria and, in a preferred embodiment, further comprises an alkaline metal selected from Na, K, and Cs. In this case, in a further preferred embodiment, the nickel content ranges from 10 to 20 wt% with respect to the whole composition of the catalyst and a cerium content from 15 to 30 wt%.

In all the cases, the nickel based catalysts are usually reduced under hydrogen flow in the range of 350°C to 500°C.

The selected catalyst is placed between the electrodes of the DBD reactor allowing the gas species to flow and is activated by high voltage (in the range of few tens of kV) pulsed discharges with a duration in the range from nanosecond to microsecond. This polarization, combined with the adsorption, desorption and catalyst/gas interaction, gives rise to the plasma formation as well as to its activation.

The activation of the catalyst by the plasma is the key of the process. The electrical power supplied to the fixed bed as a sinusoidal high voltage or a pulsed one, creates multiple streamers on the carrier HV (high voltage) waves, during the positive and negative polarization. These streamers from hundreds of picoseconds to tenths of nanoseconds, corresponding at a frequency of a few MHz, are responsible for the negative or positive polarization of the catalyst sites. This polarization induces adsorption and desorption reactions even at low temperatures (<200°C). Without polarization (conventional process) the working temperature is higher, typically, from 300°C to 420°C. In a further embodiment, the catalyst of the invention is activated by a DBD plasma by supplying an electrical power of 3 W/g of catalyst to the reaction bed.

Preferably, the process is carried out at adiabatic conditions conditions until a steady state is reached continuing then with isothermal conditions.

Under adiabatic conditions (figure 2), with plasma, the process starts at room temperature; then the discharge increases the temperature. The reaction then starts and due to its exothermicity, reactor temperature further increases and is stabilized, below 150°C depending on the thermal conductivity of the overall system.

Under isothermal conditions (figure 3), in the range 200°C-420°C, the reactor is heated by an external heat source in both cases (plasma or without plasma). This range of temperature was explored because 360°C is the optimum for the Sabatier reaction in the conventional process disclosed in the state of the art.

Furthermore, a reactor suitable for carrying out the process according to the present invention is disclosed. Said reactor according to figure 1, which is used only with illustrative purposes, comprises the following elements:
a) a cylindrical tube 1 of glass or silica, with an external diameter which can range from 8 to 20 mm (present diameter 10 mm), and a thickness wall between 1 and 2 mm, which is the reactor wall. The external face of the tube, in the central part is surrounded by a metal foil 2 which constitutes the ground electrode.
b) a second concentric tube 3, glass, silica, alumina or other dielectric materials, with a diameter ranging from of 3 to 16 mm (present diameter 3 mm). This tube constitutes the envelope of the high voltage electrode 4.
c) the catalyst 5, mesoporous ceria-zirconia or enriched zeolite, fills the annular space between the two electrodes on the central part of the reactor. In the present configuration the catalyst volume is 0.5 ml but may be increased to 5 ml by changing the diameters of the two concentric tubes.

The high voltage electrode 4 can be supplied by an AC or pulsed current with a voltage of 8-15 kV (14 kV in the present conditions). The power injected in these conditions is about 3 W per g of catalyst.

The feed of gas in the inlet 6a with a composition of 20 vol.% CO₂ and 80 vol.% H₂ has a total flow of 200 cm³/min. The reactor can be heated by hot air until 500°C.

The gas composition of the products, at the outlet of the reactor 6b, is analised by gas chromatography, IR spectroscopy, mass spectrometry and by a specific sensor for COₓ. The condensable products, mainly water, are collected for further analysis.

The mass balance is performed at steady state (after 20 min of operation), and the conversion rate of carbon dioxide and the methane or carbon monoxide production is calculated.

The main goal, under plasma conditions, is to reach an "athermal" reactor, meaning that the temperature is close to the one resulting from the energy balance between gas heating, enthalpy production and loss of energy through the quartz tube. Thus, the temperature of the reactor is stabilized, under plasma conditions, at a very low range, less than 150°C. Nevertheless, experiments have also been performed at 200, 320, 370, 420, 500°C in order to verify the overall characteristics of these performances.

### EXAMPLES

The present invention is further disclosed by way of examples which only intend to illustrate the present invention and by no means are limiting the scope thereof.

### Example 1

A typical reactor operation and procedure as disclosed in the present invention is as follows:
With 300 mg of catalyst based on mesoporous Ceria-Zirconia as supporting material corresponding to an GHSV (gas hourly space velocity) of 20,000 h⁻¹, the reactor is supplied with a mixture of carbon dioxide and hydrogen (20% vol CO₂) with a total flow rate of 12,000 ml/h STP (standard temperature and pressure) (2,400 ml/h STP CO₂ and 9,600 ml/h STP H₂), at atmospheric pressure and at temperature of 20°C.
Without plasma, no change in the composition of outlet gases is observed. When the plasma is switched on, temperature increases slowly to 150°C and after stabilization a production of methane and liquid water is obtained (after condensation) at the outlet.
Typical composition of products at the outlet is: 550 ml/h STP CO₂, 2,190 ml/h STP H₂, 1,800 ml/h STP CH₄ and 2.9 ml/h of liquid H₂O, corresponding to 3,600 ml/h of vapor H₂O. Total conversion of CO₂ in this case is 77% and the selectivity toward CH₄ formation is 100%.

When the plasma is switched off and the reactor is maintained at 150°C by an external heating, the conversion of CO₂ is under 1 % (within experimental error).

### Example 2

Another catalytic plasma reactor operation and procedure as disclosed in the present invention is as follows:
With 300 mg of catalyst based on a Ni and ceria containing zeolite, corresponding to an GHSV (gas hourly space velocity) of 20,000 h⁻¹, the reactor is supplied with a mixture of carbon dioxide and hydrogen (20% vol CO₂) with a total flow rate of 12,000 ml/h STP (standard temperature and pressure) (2,400 ml/h STP CO₂ and 9,600 ml/h STP H₂), at atmospheric pressure and at temperature of 20°C.
Without plasma, no change in the composition of outlet gases is observed. When the plasma is switched on, temperature increases slowly up to around 120°C and after stabilization a production of methane and liquid water is obtained (after condensation) at the outlet.
Typical composition of products at the outlet is: 720 ml/h STP CO₂, 2,460 ml/h STP H₂, 1,500 ml/h STP CH₄ and 2.4 ml/h of liquid H₂O, corresponding to 3,000 ml/h of vapor H₂O. Total conversion of CO₂ in this case is 63% and the selectivity toward CH₄ formation is 94% (103 ml/h STP CO).

When the plasma is switched off and the reactor is maintained at 120°C by an external heating, the conversion of CO₂ is under 1% (within experimental error).

In both examples, during the elementary pulses due to the applied high frequency streamers an activation of the sites is obtained. It increases the desorption rate of H₂O which leads to an increase of the global conversion rate of CO₂ at low temperature (<150°C) and an increase simultaneously of the time life of the catalyst. Moreover, the breakthrough of the DBD plasma use is an increasing of the conversion rate of CO₂ to CH₄ in all cases, using Ni supported on mesoporous ceria or ceria/zirconia or alternatively Ni/Ce zeolite based catalyst for a range of temperature between 320 to 420 °C.

What is remarkable in the process of the invention is that, at low temperature range, between 140°C-150°C, the conversion jumps from 1% (catalyst without discharge) to above 78% (catalyst + plasma discharge), under adiabatic conditions (fig. 2).

When the reactor works in adiabatic conditions, which means that the initial temperature when the process starts is room temperature, after the discharge the temperature is increased about 20°C. As the reaction proceeds and, due to its exothermicity, the temperature further increases and it is stabilized at around 140-150°C.

At the end of the experiment, the plasma is switched off and the reactor is heated, by an external heat source, in order to keep the temperature at the same range.

In the range of 220°C - 420°C the reactor usually works in isothermal conditions (the reactor is heated by external heat source) and a better conversion of CO₂ for the case of plasma+catalyst when comparing to catalyst alone is observed. A higher conversion is achieved at "low temperature", at around 220°C. See figure 3.

The selectivity to CH₄ formation decreases at high temperature due to the competitive mechanism which takes place: CO₂ + H₂ ⇔ CO + H₂O (RWGS reaction) in this range of temperature when ceria-zirconia support is used. This effect is not observed for pure ceria. A further advantage of the instant process is that the deposition of carbon on the catalyst that could take place during reduction of CH₄ to carbon is avoided.

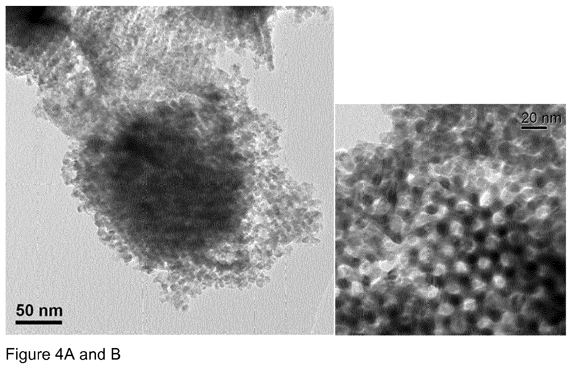

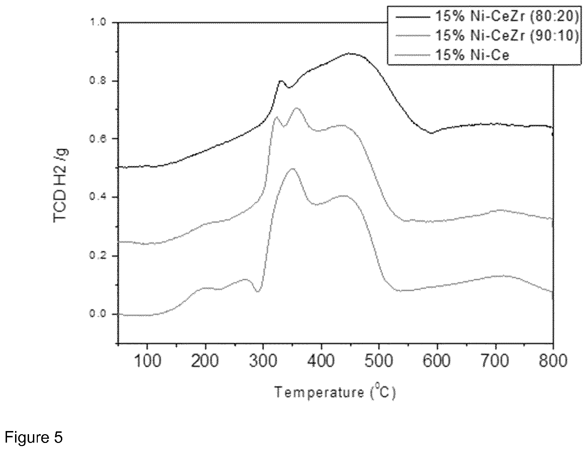

Figures 4 and 5 show in detail the catalysts used in the present invention. Figures 4A and B point out details about the mesoporous size of the ceria zirconia porous wall as well as of the porous one. Figure 5 corresponds to the hydrogen consumption pointing out the existence of surface sites with very low activation energy.

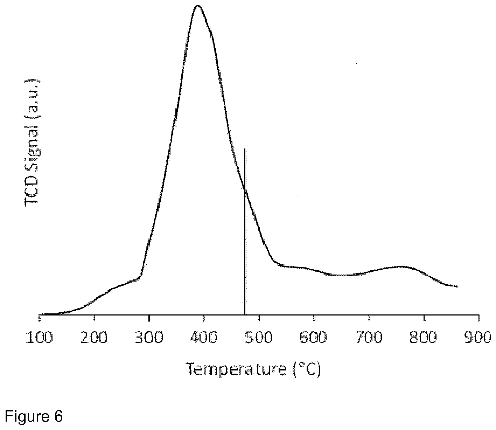

Figure 6 corresponds to the hydrogen consumption of a Ni zeolite based catalyst under H₂-TPR conditions, pointing out that, after reductive pre-treatment, most of the Ni particles are reduced and consequently showing a very low activation energy.

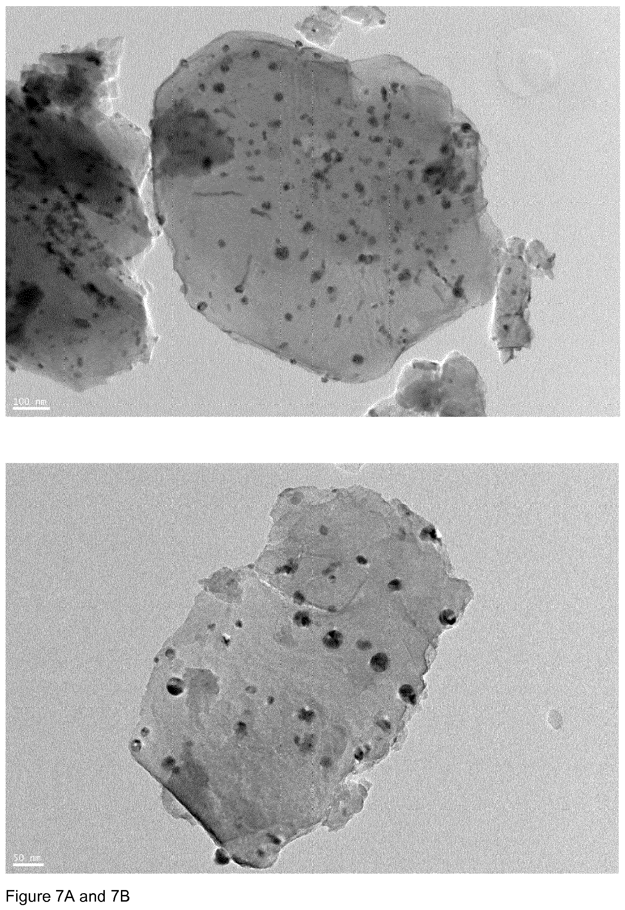

Figure 7A and 7B show TEM micrographs of a Ni/zeolite catalysts.

## Claims

1. Process for performing the following reaction with a catalyst:
CO₂ + 4 H₂ -> CH₄ + 2 H₂O
**characterized in that**:
- said catalyst is based on the combination of ceria and nickel; and
- said catalyst is activated by a DBD (dielectric barrier discharge) plasma.

2. Process according to claim 1, **characterized in that** said catalyst based on the combination of ceria and nickel is obtained from a mesoporous ceria or ceria-zirconia enriched with nickel.

3. Process according to claim 1, **characterized in that** said catalyst based on the combination of ceria and nickel is obtained from a zeolite impregnated with nickel and ceria.

4. Process according to claim 2, **characterized in that** said mesoporous ceria or ceria-zirconia enriched with nickel further comprises rare-earth oxides.

5. Process according to any of claims 2 or 4, **characterized in that** said mesoporous ceria or ceria-zirconia enriched with nickel has a content of nickel between 5 and 15 wt%.

6. Process according to any of claims 2, 4 or 5, **characterized in that** said mesoporous ceria-zirconia enriched with nickel has a content of zirconia up to 20 wt%.

7. Process according to claim 3, **characterized in that** said zeolite impregnated with nickel and ceria further comprises an alkaline metal selected from Na, K, and Cs.

8. Process according to any of claims 3 or 7, **characterized in that** said zeolite impregnated with nickel and ceria has a content of nickel from 10 to 20 wt% and cerium from 15 to 30 wt%.

9. Process according to any of the preceding claims, **characterized in that** said catalyst is activated by a DBD plasma by supplying an electrical power of 3 W/g of catalyst to the reaction bed.

10. Process according to any of the preceding claims, **characterized in that** said process is performed at adiabatic conditions until a steady state is reached continuing then with isothermal conditions.

## Patentansprüche

1. Verfahren zum Durchführen der folgenden Reaktion mit einem Katalysator:
CO₂ + 4 H₂ -> CH₄ + 2 H₂O
**dadurch gekennzeichnet, dass**
- der Katalysator auf der Kombination von Ceroxid und Nickel basiert ist; und
- der Katalysator durch ein DBD (dielektrische Barriereentladung) Plasma aktiviert wird.

2. Verfahren nach Anspruch 1, **dadurch gekennzeichnet, dass** der Katalysator, der auf der Kombination von Ceroxid und Nickel basiert, von mesoporösem Ceroxid oder Ceroxid-Zirkoniumoxid, angereichert mit Nickel, erhalten wird.

3. Verfahren nach Anspruch 1, **dadurch gekennzeichnet, dass** der Katalysator, der auf der Kombination von Ceroxid und Nickel basiert, von einem mit Nickel und Ceroxid imprägnierten Zeolithen erhalten wird.

4. Verfahren nach Anspruch 2, **dadurch gekennzeichnet, dass** das mesoporöse Ceroxid oder Ceroxid-Zirkoniumoxid, angereichert mit Nickel, weiter seltene Erdenoxide umfasst.

5. Verfahren nach einem der Ansprüche 2 oder 4, **dadurch gekennzeichnet, dass** das mesoporöse Ceroxid oder Ceroxid-Zirkoniumdioxid, angereichert mit Nickel, einen Gehalt an Nickel zwischen 5 und 15 Gew.-% aufweist.

6. Verfahren nach einem der Ansprüche 2, 4 oder 5, **dadurch gekennzeichnet, dass** das mit Nickel angereicherte mesoporöse Ceroxid-Zirkoniumoxid einen Gehalt an Zirkoniumoxid von bis zu 20 Gew.-% aufweist.

7. Verfahren nach Anspruch 3, **dadurch gekennzeichnet, dass** der mit Nickel und Ceroxid imprägnierte Zeolith weiter Alkalimetalle, die von Na, K, und Cs ausgewählt sind, umfasst.

8. Verfahren nach einem der Ansprüche 3 oder 7, **dadurch gekennzeichnet, dass** der mit Nickel oder Ceroxid imprägnierte Zeolith einen Anteil an Nickel von 10 bis 20 Gew.-% und an Cer von 15 bis 30 Gew.-% aufweist.

9. Verfahren nach einem der vorangegangenen Ansprüche, **dadurch gekennzeichnet, dass** der Katalysator durch ein DBD Plasma aktiviert wird, indem eine elektrische Leistung von 3 W/g Katalysator dem Reaktionsbett zugeführt wird.

10. Verfahren nach einem der vorangegangenen Ansprüche, **dadurch gekennzeichnet, dass** das Verfahren unter adiabatischen Bedingungen bis zum Erreichen eines Gleichgewichtszustands durchgeführt wird, wobei man dann unter isothermen Bedingungen fortfährt.

## Revendications

1. Procédé de mise en œuvre de la réaction suivante avec un catalyseur :
CO₂ + 4 H₂ -> CH₄ + 2 H₂O
**caractérisé en ce que** :
- ledit catalyseur est basé sur la combinaison d'oxyde de cérium et de nickel ; et
- ledit catalyseur est activé par un plasma DBD (décharge à barrière diélectrique).

2. Procédé selon la revendication 1, **caractérisé en ce que** ledit catalyseur basé sur la combinaison d'oxyde de cérium et de nickel est obtenu à partir d'un oxyde de cérium mésoporeux ou oxyde de cérium-oxyde de zirconium enrichi avec du nickel.

3. Procédé selon la revendication 1, **caractérisé en ce que** ledit catalyseur basé sur la combinaison d'oxyde de cérium et de nickel est obtenu à partir d'une zéolite imprégnée avec du nickel et de l'oxyde de cérium.

4. Procédé selon la revendication 2, **caractérisé en ce que** ledit oxyde de cérium mésoporeux ou oxyde de cérium-oxyde de zirconium enrichi avec du nickel comprend en outre des oxydes de terres rares.

5. Procédé selon l'une quelconque des revendications 2 ou 4, **caractérisé en ce que** ledit oxyde de cérium mésoporeux ou oxyde de cérium-oxyde de zirconium enrichi avec du nickel a une teneur en nickel comprise entre 5 et 15 % en poids.

6. Procédé selon l'une quelconque des revendications 2, 4 ou 5, **caractérisé en ce que** ledit oxyde de cérium mésoporeux-oxyde de zirconium enrichi avec du nickel a une teneur en nickel allant jusqu'à 20 % en poids.

7. Procédé selon la revendication 3, **caractérisé en ce que** ladite zéolite imprégnée avec du nickel et de l'oxyde de cérium comprend en outre un métal alcalin choisi parmi Na, K et Cs.

8. Procédé selon l'une quelconque des revendications 3 ou 7, **caractérisé en ce que** ladite zéolite imprégnée avec du nickel et de l'oxyde de cérium a une teneur en nickel de 10 à 20 % en poids et en oxyde de cérium de 15 à 30 % en poids.

9. Procédé selon l'une quelconque des revendications précédentes, **caractérisé en ce que** ledit catalyseur est activé par un plasma DBD en fournissant une alimentation électrique de 3 W/g de catalyseur au lit réactionnel.

10. Procédé selon 'une quelconque des revendications précédentes, **caractérisé en ce que** ledit procédé est mis en œuvre dans des conditions adiabatiques jusqu'à ce qu'un état stable soit atteint en poursuivant ensuite avec des conditions isothermes.
